# NEUE EUROPÄISCHE PATENTSCHRIFT

(11) **EP 2 371 525 B2**
(45) Veröffentlichungstag und Bekanntmachung des Hinweises auf die Entscheidung über den Einspruch: **03.01.2018**
(45) Hinweis auf die Patenterteilung: 12.02.2014
(21) Anmeldenummer: 11160242.1
(22) Anmeldetag: 29.03.2011
(51) Int. Cl.: B30B 15/08, C12M 1/107

(54) **Doppelschneckenextruder und Verfahren zum thermomechanischen Aufschluss von organischen Roh- und Reststoffen**
Twin screw extruder and method for the thermo-mechanical pulping of organic raw and residual materials
Extrudeuse à deux vis et procédé de fusion thermomécanique de matières brutes et de résidus organiques

(30) Priorität: 01.04.2010 DE 102010003601
(43) Veröffentlichungstag der Anmeldung: 05.10.2011
(73) Patentinhaber: Börner, Frank, 01468 Friedewald (DE); Börner, Jürgen, 01468 Friedewald (DE)
(72) Erfinder: Börner, Frank, 01468 Friedewald (DE); Börner, Jürgen, 01468 Friedewald (DE)
(74) Vertreter: Sperling, Thomas

(56) Entgegenhaltungen:
- EP-A1- 1 978 086
- WO-A1-84/00378
- WO-A1-98/41646
- DE-A1- 1 928 676
- DE-A1- 4 021 550
- DE-A1- 4 423 099
- DE-A1-102004 054 468
- DE-B1- 2 423 785
- DE-U1-202008 000 161
- GB-A- 791 234
- GB-A- 2 304 619
- JP-A- 2006 239 729
- DATABASE WPI Week 198542 Thomson Scientific, London, GB; AN 1985-261155 XP002662448, -& JP 60 176556 A (MITSUBISHI KAKOKI KAISHA) 10. September 1985 (1985-09-10)
- DATABASE WPI Week 198721 Thomson Scientific, London, GB; AN 1987-146766 XP002662449, -& JP 62 083857 A (SHOKUHIN SANGYO EXTRUSION) 17. April 1987 (1987-04-17)
- Lehmann Maschinenbau GmbH, Auszug "Betriebsanleitung für den Extruder MSZ-B74e",Deckblatt, Seiten 2,4 und eine Zeichnung "Ersatz- und Verschleissteile".
- Lehmann Maschinenbau GmbH, zwei Zeichnungen zu MSZK-B74e.
- Lehmann Maschinenbau GmbH: 6 Rechnung datiert zwischen 08.2007 bis 07.2009 für die Anzahlung oder Lieferung des Extruders B47e.
- Lehmann Maschinenbau GmbH, Bestätigung für Auftrag-Nr. 20000400 vom 14.06.2000 über einen Extruder MSZ-T 15/2 für die TU Dresden, Institut für Holz- und Papiertechnik.
- Einzelfotografie: "Gesamtansicht MSZ-T 15/2" laut Angabe im Einspruchsschreiben vom 12.11.2014
- Einzelfotografie: Typenschild MSZ-T 15/2
- Einzelfotografie: "Verschleissseinlage, Gehäuse" laut Angabe im Einspruchsschreiben vom 12.11.2014
- Einzelfotografie: "Draufsicht auf das freie Ende der Extruderschnecken im Endmodul mit gegenläufiger Schneckensteigung" laut Angabe im Einspruchsschreiben vom 12.11.2014
- Einzelfotografie: "Vorderansicht mit Antrieb der beiden Extruderschnecken" laut Einspruchsschreiben vom 12.11.2014
- Pfriem, Diplomarbeit, TU Dresden, 24.06.2003, Seite 32 (A.2.7. a); sowie Pfriem,Diplomarbeit, TU Dresden, 24.06.2003, Seiten 4-7, 24-32, und 115-117 (A.2.7.b)
- NGUYEN T. CONG. ET AL.: 'Alternatives Verfahren zur Zerfaserung von Einjahrespflanzen für klein- und mittelständische Unternehmen' HOLZTECHNOLOGIE Bd. 47, Nr. 4, 2006, Seiten 11 - 15
- Anlage 3.1.2 Seiten mit 4 Fotografien, überschrieben mit "MSEK 200-TU Freiberg 1997"
- Schubert, Dissertation, TU Freiberg, 14.10.2003, Anlage 1: "Aufbau des Doppelschneckenextruders der Fa. Lehmann Maschinenbau GmbH"
- Warnecke, Dissertation, TU Freiberg, 22.09.2006, Seiten 41-42
- Einzelfotografie: Typenschild MSZ-T 15/3
- Einzelfotografie: "Draufsicht auf Extruderschnecken" laut Einspruchsschreiben vom 12.11.2014
- Einzelfotografie: "Draufsicht auf Verschleisseinlage des Gehäusedeckels mit abgewinkelten Flanschen" laut Einspruchsschreiben vom 12.11.2014
- Einzelfotografie: "Seitenansicht Extruder mit geöffnetem Gehäusedeckel" laut Einspruchsschreiben vom 12.11.2014
- Lehmann Maschinenbau GmbH, Zeichnung "Verschleisseinlage unten".
- Einzelfotografie: "Bei der Firma Agrofarm 2000 GmbH eingebauter Extruder MSZK 90e" laut Einspruchsschreiben vom 12.11.2014
- Einzelfotografie: "Gesamtansicht Extruder MSZK 90e" laut Einspruchsschreiben vom12.11.2014
- Einzelfotografie: "Aufgeklappter Gehäusedeckel mit Verschleisseinlage mit abgekanteten Flanschen" laut Einspruchsschreiben vom 12.11.2014
- Einzelfotografie:"Vorderansicht Extruder ohne Stirnplatte" laut Einspruchsschreiben vom 12.11.2014
- 2 Seiten mit 4 Fotografien: "Aufnahmen des Herstellers" laut Einspruchsschreibenvom 12.11.2014
- Lehmann Maschinenbau GmbH, Auszug "Betriebsanleitung Anlagentechnik zur Zerfaserung biogener Stoffe für den Betreiber Agrofarm 2000 GmbH", Seite 12.
- Englische Übersetzung der Absätze von JP 2006239729 A, 21 Seiten
- Abbildung 4 aus dem Streitpatent EP-A-2371525
- Lehmann Maschinenbau GmbH:"Biogasanlagenbau - auf den Aufschluss kommt es an", Biogastagung Innovationskongress Osnabrück am 12.06.2008, 4 Seiten
- Lehmann Maschinenbau GmbH:"Aufschluss von Biomasse sichert hohen Biogasertrag", enertec 2009, 28.01.2009, 7 Seiten
- Erklärung von Frau Katharina Büttner vom 07.04.2016, sowie Auszug aus dem Handelregister des Amtsgerichts Chemnitz vom 07.04.2016

## Beschreibung

Die Erfindung betrifft einen Doppelschneckenextruder zum thermomechanischen Aufschluss von organischen Roh- und Reststoffen. Des Weiteren betrifft die Erfindung ein mit dem erfindungsgemäßen Doppelschneckenextruder durchgeführtes Verfahren zum thermomechanischen Aufschluss von organischen Roh- und Reststoffen.

Das Hauptanwendungsgebiet der Erfindung ist der thermomechanische Aufschluss von biologischen Nutz- und Restwertstoffen, wie Mais, Getreide, Gras, Mist, Holz, Rüben, Obst, Gemüse und Gartenabfällen für die Biogasgewinnung. Des Weiteren kann die Erfindung auch für die Futteraufbereitung und Futtermischung in der Tierhaltung sowie zur Aufspaltung von Hülsenfrüchten für die Pflanzenölgewinnung verwendet werden. Ebenso ist der Einsatz der Erfindung nicht nur beim Aufschluss von nachwachsenden Rohstoffen, sondern auch bei der Aufbereitung von feststoffartigen fossilen Rohstoffen oder von anorganischen Materialien möglich beziehungsweise denkbar.

Bei zahlreichen Aufschlussverfahren, wie zum Beispiel für den Aufschluss von Biomasse, insbesondere biologischen Nutz- und Restwertstoffen oder Holz, sowie für den Aufschluss von Kunststoffen, werden bereits parallellaufende Doppelschneckenextruder verwendet.

In der EP 1 978 086 A1 sind ein Verfahren und eine geeignete Vorrichtung zum Hybridaufschluss von lignocellulosehaltiger Biomasse durch Zerfaserung und Hydrolyse mit der Gerätekombination bekannter Zerfaserungsgeräte, wie zum Beispiel Extruder, und einer Hochdruckmikrowelle zur Nutzung in Biogas- und Bioethanol-Produktionsprozessen beschrieben.

Aus der DE 10 2004 054 468 A1 ist ein Verfahren zur anaeroben Vergärung pflanzlicher Substrate in einem Reaktor und deren Vorbehandlung mittels thermomechanischem Aufschluss bekannt. Zur Erzielung einer höheren Gasbildungsrate wird dem Vergärungsprozess eine aufbereitungstechnische Vorstufe außerhalb des Reaktors vorgeschaltet, in der eine Extrusion der pflanzlichen Substrate stattfindet. Durch die Extrusion entsteht eine hochgradig homogenisierte Biomasse, deren Zellgefüge weitgehend zerstört ist. Durch die Zerkleinerung des biogenen Materials und die dadurch erzielte Oberflächenvergrößerung wird eine schnellere Nährstoffverfügbarkeit für die Methanbildner ermöglicht.

Bei diesem Verfahren wird in dem Extruder das Substrat mehrfach stark zusammengepresst und wieder entspannt. Durch den hohen Druck erhitzt sich das im Substrat enthaltene Wasser und verdampft bei Entspannung explosionsartig. Dadurch wird die Zellstruktur der Inputstoffe von innen heraus aufgerissen. Auf diese Weise wird den Bakterienkulturen, welche in der Hydrolyse diese Funktion normalerweise übernehmen, der Zugang zu den Nährstoffen wesentlich erleichtert. Der Hydrolyseprozess und damit der gesamte Abbauprozess organischer Masse zu Biogas wird somit verkürzt. Zudem hat sich in umfangreichen Vergärungsversuchen gezeigt, dass sich die Biogasausbeute insgesamt erhöht, da das Verfahren auch Zellbestandteile des Substrats aufbricht, die die Hydrolysebakterien nicht oder nur sehr schwer aufbrechen können.

Zusammenfassend ergeben sich durch ein thermomechanisches Aufschlussverfahren unter Einsatz eines Doppelextruders in bereits bestehenden Biogasanlagen Vorteile, welche im Folgenden dargestellt sind. Die erforderlichen Substratmengen und die damit verbundenen Substratkosten werden reduziert. Es werden Stoffbestandteile aufgeschlossen, die bisher nur schwer vergoren werden konnten. Dadurch erhöht sich die spezifische Gasausbeute je nach Substrat um 15 bis 25 Prozent.

Des Weiteren werden die Elektroenergiekosten für die Rührtechnik reduziert. Da sich durch den Voraufschluss im Bioextruder später im Fermenter kaum Schwimmschichten bilden können, ist eine Verringerung der Rührzeiten und damit des Elektroenergiebedarfs der Rührwerke möglich.

Durch die Anwendung des thermomechanischen Aufschlussverfahrens wird die Palette der vergärbaren Substrate vergrößert. Dadurch ist auch der Einsatz von nachwachsenden Rohstoffen, die preisgünstiger als die bisher verwendeten sind, möglich, insbesondere von landwirtschaftlichen Reststoffen wie Mais- und Getreidestroh.

Darüber kann aufgrund der stabileren Prozessbiologie die Jahresauslastung der Biogasanlage erhöht werden. Durch die Verkürzung der Abbauzeiten wird die Raumbelastung im Fermenter gesenkt, was einen stabileren Prozess zur Folge hat. Im Doppelschneckenextruder wird das Substrat auf circa 70 °C erhitzt, was zu einer Verringerung von unerwünschten Pilzkulturen (Fusarien) führt.

Die im Folgenden genannten Vorteile ergeben sich durch den Einsatz des Doppelschneckenextruders in neu zu errichtenden Biogasanlagen.

Die Einnahmen für erzeugte Elektroenergie und Wärme können erhöht werden. Dadurch, dass Stoffbestandteile aufgeschlossen werden, die bisher nur schwer vergoren werden konnten, und dass sich die spezifische Gasausbeute je nach Substrat gegenüber herkömmlichen Anlagen um 15 bis 25 Prozent erhöht, kann mehr Energie aus den vorhandenen Substraten gewonnen werden.

Die Nachteile der thermomechanischen Aufschlussverfahren des Standes der Technik bestehen in kurzen Standzeiten von Schnecken und Verschleißblechen. Im Ergebnis dessen entstehen hohe Betriebskosten. Außerdem sind diese Verfahren immer noch mit einem sehr hohen Energieverbrauch verbunden. Ein weiterer Nachteil besteht in einem erschwerten Zugang zu Anlage für die Wartung und Reparatur. Schließlich können die Inputstoffe in die bestehenden Anlagen nicht kontrolliert zugeführt werden.

Aus der GB 2 304 619 A ist eine Extrusionsvorrichtung für Doppelschnecken mit Verschleißeinlagen bekannt, die einen W-förmigen Querschnitt aufweisen. Diese Einlagen weisen Abkantungen an den äußeren Rändern auf. Jeweils zwei der W-förmigen Einlagen bilden spiegelsymmetrisch aneinanderliegend zwei längliche parallele Hohlräume für die Aufnahme der Extruder-Doppelschnecke.

Die Aufgabe der Erfindung besteht nicht nur im Bereitstellen einer Vorrichtung, bei der die Standzeiten für die Verschleißteile gegenüber bisherigen Anlagen erhöht sind. Darüber hinaus soll mithilfe dieser Vorrichtung die Effizienz beim mechanischen Aufschluss von Biomasse und damit der Vergrößerung der Oberfläche des biogenen Materials erhöht werden. Auf diese Weise soll es mit der Vorrichtung möglich sein, den Gasertrag bei der anaeroben Vergärung von Biomasse im Vergleich zu den Anlagen aus dem Stand der Technik weiter zu erhöhen. Des Weiteren sollen mithilfe der Vorrichtung die Verweilzeit der Biomasse im Fermenter und der Energieverbrauch gesenkt sowie größere Fördermengen erreicht werden können. Des Weiteren sollen die Zugangsmöglichkeiten zur Anlage zwecks Wartung verbessert werden.

Die Lösung der Aufgabe der Erfindung liegt in der Bereitstellung eines Doppelschneckenextruders zum thermomechanischen Aufschluss von organischen Roh- und Reststoffen, umfassend
- ein längliches Extrudergehäuse, aufweisend ein Gehäuseunterteil mit einem auf der Innenseite U-schalenförmigen Querschnitt mit nach oben gerichteter Öffnung, das sich horizontal zwischen einer hinteren Stirnplatte und einer vorderen Stirnplatte erstreckt, wobei das Gehäuseunterteil in einem Schließzustand zusammen mit einem Gehäusedeckel, der einen auf der Innenseite umgedreht U-schalenförmigen Querschnitt mit nach unten gerichteter Öffnung aufweist, durch Übereinanderlegen der Öffnung des Gehäuseunterteils und der Öffnung des Gehäusedeckels einen hohlzylinderartigen Führungsabschnitt bildet, der an der vorderen Stirnplatte anliegt,
- zwei nebeneinander parallel angeordnete, gegenläufige Extruderschnecken, die beide, jeweils freitragend, nur an einem ersten Ende an der hinteren Stirnplatte des länglichen Extrudergehäuses gelagert sind und am gegenüberliegenden zweiten Ende jeweils ein nichtgelagertes, freies Endmodul aufweisen, wobei die Extruderschnecken im Schließzustand zumindest teilweise vom hohlzylinderförmigen Führungsabschnitt aus dem Gehäuseunterteil und dem Gehäusedeckel umschlossen sind.

Erfindungsgemäß sind das Gehäuseunterteil und der Gehäusedeckel auf ihren Innenseiten jeweils mit Verschleißeinlagen versehen, wobei die Verschleißeinlage des Gehäuseunterteils einen U-schalenförmigen Querschnitt mit nach oben gewölbten Seitenwandungen aufweist. Die nach oben gewölbten Seitenwandungen bilden im U-schalenförmigen Querschnitt die U-Schenkel. An ihren oberen Enden, die sich an der oberen Öffnung des Gehäuseunterteils befinden, sind die nach oben gewölbten Seitenwandungen des Gehäuseunterteils jeweils unter Bildung von Flanschen nach außen horizontal abgekantet. Die Verschleißeinlage des Gehäusedeckels bildet einen im Schließzustand umgedreht U-schalenförmigen Querschnitt mit zwei nach unten gewölbten Seitenwandungen, die bei dem im Schließzustand umgedreht U-schalenförmigen Querschnitt die U-Schenkel bilden. An den unteren Enden der nach unten gewölbten Seitenwandungen sind diese unter Bildung von Flanschen ebenfalls nach außen horizontal abgekantet. Im Schließzustand, beim Übereinanderlegen der Öffnungen des Gehäuseunterteils und des Gehäusedeckels, kontaktieren die beiden nach außen abgekanteten Flansche des Gehäuseunterteils und die nach außen abgekanteten Flansche des Gehäusedeckels miteinander. Erfindungsgemäß weisen die Extruderschnecken jeweils eine Schneckensteigung auf, die vom ersten Ende bis zum Beginn des freien Endmoduls immer kleiner wird, wobei das freie Endmodul eine gegenläufige Schneckensteigung aufweist. Eine kleinere Schneckensteigung ist gleichbedeutend mit einem kleineren Abstand zwischen den Stegen der Schneckenwendel.

Die aus der GB 2 304 619 A an sich bekannte und beim erfindungsgemäßen Doppelschneckenextruder ebenfalls vorgesehene Abkantung der Verschleißeinlagen in den Außenbereichen unter Bildung von Flanschen, die in Schließstellung des länglichen Extrudergehäuses miteinander kontaktieren, hat den Vorteil, dass das extrudierte Gut nicht hinter die Verschleißeinlagen, das heißt zwischen die Verschleißeinlagen und die Gehäusewände des Gehäuseunterteils und des Gehäusedeckels gelangen kann, was eine Knickbewegung der Verschleißbleche und damit eine kürzere Standzeit der Verschleißbleche und der Extruderschnecken zur Folge hätte. Die erreichte Standzeit beträgt überraschenderweise bis zum Vierfachen dessen, was in herkömmlichen Anlagen an Standzeit erreicht wurde.

Die wechselnde Schneckensteigung hat zur Folge, dass die Effizienz bei der Zerfaserung verbessert wird. Da die Schnecken von ihrer Steigung her zum Ende zu immer kleiner werden und das Endmodul eine gegenläufige Steigung aufweist, entsteht bei der Extrusion ein Gegendruck, der zur Verbesserung des Zerfaserungsergebnisses beiträgt.

Beide Maßnahmen, sowohl die konstruktive Gestaltung der Verschleißeinlagen als auch die der Extruderschnecken, führen im Ergebnis zu einer signifikanten Erhöhung des Durchlaufvolumens an zu extrudierendem Material.

Gemäß einer bevorzugten Ausführungsform der Erfindung weist die Verschleißeinlage des Gehäuseunterteils eine flache Bodenwandung auf, die im Querschnitt den U-Basisbereich bildet. Vorzugsweise weist die Verschleißeinlage des Gehäusedeckels eine flache Deckwandung auf, die im umgedreht U-schalenförmigen Querschnitt den U-Basisbereich bildet. Dabei sind die flache Deckwandung und die flache Bodenwandung im Schließzustand parallel beabstandet. In einer vorteilhaften Ausgestaltung dieser Ausführungsform ist auf der flachen Bodenwandung der Verschleißeinlage des Gehäuseunterteils und auf der flachen Deckwandung der Verschleißeinlage des Gehäusedeckels jeweils ein Führungsprisma ausgebildet, das im Querschnitt jeweils die Form eines dreieckigen, jeweils in der Mitte der Bodenwandung und der Deckwandung platzierten Vorsprungs mit konkav gekrümmten Schenkeln aufweist und sich über die gesamte Länge der jeweiligen Verschleißeinlage erstreckt. Die konkav gekrümmten Schenkel der gegenüberliegenden Führungsprismen auf der Bodenwandung und der Deckwandung sowie die gekrümmten Seitenwandungen bilden zusammen in vorteilhafter Weise jeweils Teilführungen für beide Extruderschnecken. Beim Extrusionsprozess erfolgt im Spalt zwischen Verschleißblech und Schneckenmantel der Extruderschnecken ein Aufspalten des zu extrudierenden Materials. Dabei dienen die Teilführungen vor allem dem Ausgleich von radialen Kräften, die beim Extrusionsprozess auf die freien Enden der Extruderschnecken wirken, und halten die Extruderschnecken in definierter Lage.

Sowohl für die Extruderschnecken als auch für die Verschleißbleche können allgemein Materialien verwendet werden, welche aus dem Bergbau oder der Kunststoffindustrie erhältlich sind. Vorteilhafterweise bestehen die Verschleißeinlagen aus einem weichen Trägerblech, das mit einer Härteschicht aus hochverschleißfestem Verbundpanzermaterial überzogen ist. Das Verbundpanzermaterial besteht bevorzugt aus einer austenitischen Stahlmatrix, in der die zum Verschleißschutz notwendigen extrem harten Carbide gleichmäßig verteilt sind.

Gemäß einer weiteren vorteilhaften Ausführungsform der Erfindung ist auf dem Gehäuseunterteil in einem Bereich, der sich zwischen der hinteren Stirnplatte und dem Gehäusedeckel erstreckt, ein Gehäuseoberteil mit einem Einlauftrichter mit einer oberen Eintrittsöffnung und einer unteren Austrittsöffnung vorgesehen, wobei sich die Austrittsöffnung des Einlauftrichters oberhalb der Extruderschnecken befindet und auf diese gerichtet ist. Dadurch kann das zu extrudierende Material direkt auf die Extruderschnecken gegeben werden.

Vorzugsweise ist zwischen dem Einlauftrichter und dem Gehäusedeckel ein Bereich vorgesehen, bei dem die Extruderschnecken mit einer vom Einlauftrichter bis zum Gehäusedeckel abfallenden Einlaufschräge überdacht sind. Infolge des Vorhandenseins der Einlaufschräge gelangt das zu extrudierende Material harmonischer in die Extruderschnecken als es bei Vorhandensein einer waagerechten Wand zwischen Einlauftrichter und Gehäusedeckel der Fall wäre, wo sich das Material häufig oberhalb der Schnecken staut.

Entsprechend einer weiteren bevorzugten Ausführungsform weist die vordere Stirnplatte eine größenverstellbare Auslauföffnung zur Mengendosierung des extrudierten Materials auf.

Der Doppelschneckenextruder in einer seiner oben genannten Ausführungen ist gemäß eines weiteren Aspekts der Erfindung Teil einer Vorrichtung zum thermomechanischen Aufschluss von organischen Roh- und Reststoffen, umfassend
- ein Untergestell,
- ein Antriebsmodul, das auf dem Untergestell befestigt ist, mit zwei Getriebemotoren und zwei Kardangelenkwellen zur Kraftübertragung sowie zwei mit den Kardangelenkwellen verbundenen Extruderachsen,
- den Doppelschneckenextruder, der auf dem Untergestell hinter dem Antriebsmodul angeordnet ist, wobei die Extruderschnecken des Doppelschneckenextruders durch das Antriebsmodul angetrieben werden.

Vorzugsweise weist das Untergestell, auf dem das Extrudergehäuse befestigt ist, unterhalb des Doppelschneckenextruders Zugangstüren für die Wartung auf. In einer weiteren vorteilhaften Ausgestaltung sind ein Metallabscheider und/oder ein Störstofffilter vor dem Zugang zum Einlauftrichter in der Vorrichtung integriert.

Ein weiterer Aspekt der Erfindung betrifft ein Verfahren zum thermomechanischen Aufschluss von organischen Roh- und Reststoffen, bei dem das aufzuschließende Material einem erfindungsgemäßen Doppelschneckenextruder zugeführt wird und in diesem einen Extrusionsprozess durchläuft. Dabei sind alle oben genannten Ausführungsformen des Doppelschneckenextruders und seiner Unterbringung in der beschriebenen Vorrichtung möglich. Der zuvor beschriebene Schließzustand des Doppelschneckenextruders liegt während des Extrusionsprozesses vor.

In einer besonders bevorzugten Ausführungsform des erfindungsgemäßen Verfahrens gelangt das zu extrudierende Material über eine externe Zuführeinrichtung in den genannten Einlauftrichter, der direkt zu den gegenläufigen Extruderschnecken führt. Gleichzeitig wird der Durchfluss des Materials über die bereits beschriebene größenverstellbare Auslassöffnung an der vorderen Stirnplatte des Doppelschneckenextruders geregelt.

Ein bevorzugtes Verwendungsgebiet des erfindungsgemäßen Doppelschneckenextruders ist ein Verfahren zum thermomechanischen Aufschluss von Biomasse als Vorbehandlung für die anaerobe Vergärung von Biomasse in einem Reaktor. Über eine externe Zuführeinrichtung gelangt die Biomasse bevorzugt in einen Einlauftrichter, der direkt zu den gegenläufigen Extruderschnecken führt, wobei die Extruderschnecken erfindungsgemäß von ihrer Steigung her zum Ende zu immer kleiner werden. Eine kleinere Schneckensteigung ist dabei gleichbedeutend mit einem kleineren Abstand zwischen den Stegen der Schneckenwendel. Das Endmodul hat eine gegenläufige Steigung, wodurch ein Gegendruck entsteht. Die Extruderschnecken haben einen geringen Spalt zueinander, sodass die Biomasse hindurchgepresst wird. Bei dem Pressen werden die einzelnen Fasern mechanisch aufgebrochen. Am Ende des Doppelschneckenextruders wird der Durchfluss gemäß einer vorteilhaften Ausführungsform über eine größenverstellbare Auslassöffnung geregelt, vorzugsweise durch die Schiebebewegung eines mit einer Schieberausnehmung formgleichen Schieberformstückes. Die Biomasse fällt dann aus der Maschine auf ein weiteres externes Transportgerät, vorzugsweise eine Pumpe, eine Schnecke oder ein Förderband, mit dem die Biomasse dann einer Fermentationsanlage zur Biogasgewinnung zugeführt werden kann.

Durch die Verwendung der erfindungsgemäßen Vorrichtung erfolgt ein effizienter mechanischer Aufschluss der Biomasse und damit eine Vergrößerung der Oberfläche. Daraus ergibt sich ein höherer Gasertrag pro vergorener Biomasse bei der anschließenden anaeroben Vergärung im Fermenter. Des Weiteren findet während des Extrusionsprozesses auch ein Vorwärmen der Biomasse statt. Aufgrund dessen werden bis circa 20 Prozent mehr Gasausbeute bei kürzerer Verweilzeit im Fermenter erreicht.

Zudem kann mehr Stoffdurchsatz in der gleichen Zeit erfolgen. Durch die Regelbarkeit der Stoffzufuhr kann auch eine gleichmäßigere Gasausbeute erreicht werden, dabei erfolgt die Regelung vorzugsweise elektrisch.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Fig. 1:**: einen Doppelschneckenextruder in einer perspektivischen Darstellung,
- **Fig. 2:**: einen Doppelschneckenextruder in der Draufsicht,
- **Fig. 3:**: eine Vorderansicht des Doppelschneckenextruders mit Auslauföffnung und
- **Fig. 4:**: eine perspektivische Darstellung einer Vorrichtung zum thermomechanischen Aufschluss von organischen Roh- und Reststoffen.

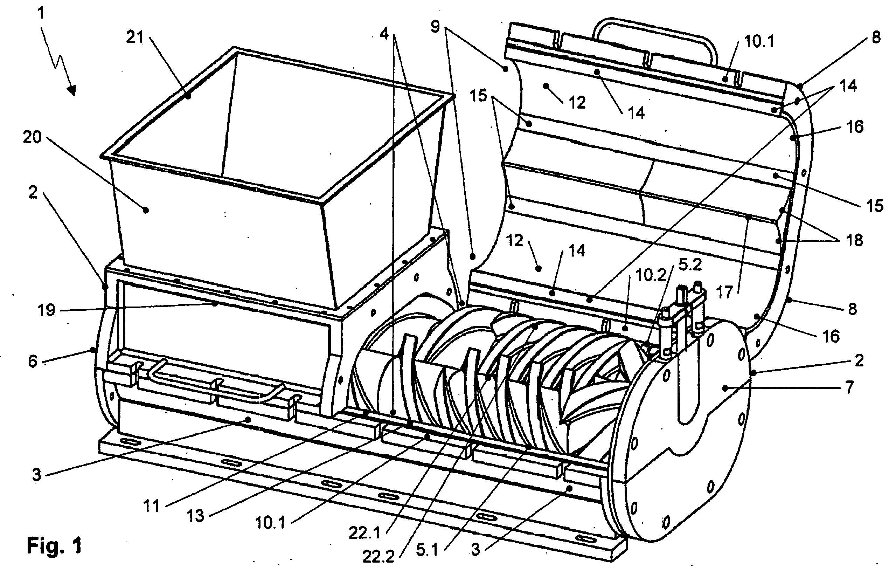

Die **Fig. 1** zeigt einen Doppelschneckenextruder 1 in einer perspektivischen Darstellung. Der Doppelschneckenextruder 1 zum thermomechanischen Aufschluss von organischen Roh- und Reststoffen umfasst ein längliches Extrudergehäuse 2, welches ein Gehäuseunterteil 3 mit einem U-schalenförmigen Querschnitt und nach oben gerichteter Öffnung 4 aufweist, wobei die Öffnung 4 durch zwei obere Längsrandflächen 5.1, 5.2 des Gehäuseunterteils 3 begrenzt wird. Das Gehäuseunterteil 3 erstreckt sich mit seinen oberen Längsrandflächen 5.1, 5.2 horizontal zwischen einer hinteren Stirnplatte 6 und einer vorderen Stirnplatte 7 des länglichen Extrudergehäuses 2. Des Weiteren ist ein Gehäusedeckel 8 vorgesehen, der einen umgedreht U-schalenförmigen Querschnitt mit nach unten gerichteter Öffnung 9 und zwei diese nach unten gerichtete Öffnung 9 begrenzende untere Längsrandflächen 10.1, 10.2 aufweist. Dabei bilden in einem Schließzustand das Gehäuseunterteil 3 und der Gehäusedeckel 8 durch Übereinanderlegen der nach oben gerichteten Öffnung 4 des Gehäuseunterteils 3 und der nach unten gerichteten Öffnung 9 des Gehäusedeckels 8, das heißt durch Übereinanderlegen der oberen Längsrandflächen 5.1, 5.2 des Gehäuseunterteils 3 und der unteren Längsrandflächen 10.1, 10.2 des Gehäusedeckels 8, zusammen einen hohlzylinderartigen Führungsabschnitt.

Der Gehäusedeckel 8 und somit auch der im Schließzustand gebildete hohlzylinderartige Führungsabschnitt aus Gehäuseunterteil 3 und Gehäusedeckel 8 grenzen an die vordere Stirnplatte 7 des länglichen Extrudergehäuses 2 an. Der Gehäusedeckel 8 ist mit einer Längsrandfläche 10.2 auf einer Längsrandfläche 5.2 des Gehäuseunterteils 3 vertikal schwenkbar gelagert, sodass der Gehäusedeckel 8 zwischen der Schließstellung und einer Öffnungsstellung bewegt werden kann, wobei die **Fig. 1** eine Öffnungsstellung zeigt. Das Gehäuseunterteil 3 und der Gehäusedeckel 8 sind auf ihren Innenseiten jeweils mit Verschleißeinlagen 11, 12 versehen, die einen jeweils U-schalenförmigen Querschnitt mit an den Öffnungen 4, 9 horizontal nach außen abgekanteten Flanschen 13, 14 aufweisen, wobei im Schließzustand beim Übereinanderlegen der Öffnungen 4, 9 die beiden nach außen abgekanteten Flansche 13 des Gehäuseunterteils 3 und die nach außen abgekanteten Flansche 14 des Gehäusedeckels 8 miteinander kontaktieren, das heißt deckungsgleich aufeinanderliegen. Die Verschleißeinlage 12 des Gehäusedeckels 8 weist eine flache Deckwandung 15 auf, die in einem im Schließzustand umgedreht U-schalenförmigen Querschnitt den U-Basisbereich bildet. Die U-Schenkel im umgedreht U-schalenförmigen Querschnitt werden durch zwei nach unten gewölbte Seitenwandungen 16 gebildet, die an ihren unteren Enden jeweils unter Bildung des Flansches 14 nach außen horizontal abgekantet sind. Die Verschleißeinlage 11 des Gehäuseunterteils 3 weist eine flache Bodenwandung auf (die in **Fig. 1** nicht sichtbar ist), die im Querschnitt den U-Basisbereich bildet. Die U-Schenkel im U-schalenförmigen Querschnitt werden durch zwei nach oben gewölbte Seitenwandungen (in **Fig.1** nicht dargestellt) gebildet, die an ihren oberen Enden jeweils unter Bildung eines Flansches 13 nach außen horizontal abgekantet sind. Dabei sind die flache Deckwandung 15 und die flache Bodenwandung im Schließzustand parallel beabstandet.

Auf der (in **Fig. 1** nicht dargestellten) flachen Bodenwandung der Verschleißeinlage 11 und auf der flachen Deckwandung 15 der Verschleißeinlage 12 ist jeweils ein Führungsprisma 17 ausgebildet, das im Querschnitt jeweils die Form eines dreieckigen, jeweils in der Mitte der Bodenwandung und der Deckwandung 15 platzierten Vorsprungs mit konkav gekrümmten Schenkeln 18 aufweist, wobei sich das Führungsprisma 17 über die gesamte Länge der jeweiligen Verschleißeinlage 11, 12 erstreckt.

Das Gehäuseunterteil 3 ist in einem Bereich, der sich zwischen der hinteren Stirnplatte 6 und dem Gehäusedeckel 8 erstreckt, mit einem Gehäuseoberteil 19 abgedeckt, wobei das Gehäuseoberteil 19 an der hinteren Stirnplatte 6 anliegt. Integraler Bestandteil des Gehäuseoberteils 19 ist ein Einlauftrichter 20 mit einer rechteckigen, oberen Eintrittsöffnung 21.

In dem länglichen Extrudergehäuse 2 sind zwei nebeneinander parallel angeordnete, gegenläufige Extruderschnecken 22.1, 22.2 gelagert. Die Extruderschnecken 22.1, 22.2 sind beide jeweils freitragend, das heißt nur an einer der Stirnplatten 6, 7 gelagert. Wie in **Fig. 1** zu sehen, sind die Extruderschnecken 22.1, 22.2 an der vorderen Stirnplatte 7, an die der hohlzylinderförmige Führungsabschnitt aus dem Gehäuseunterteil 3 und dem Gehäusedeckel 8 angrenzt, nicht gelagert. Der hohlzylinderförmige Führungsabschnitt aus dem Gehäuseunterteil 3 mit der Verschleißeinlage 11 und dem Gehäusedeckel 8 mit Verschleißeinlage 12 umschließt im Schließzustand einen Teil der zwei Extruderschnecken 22.1, 22.2.

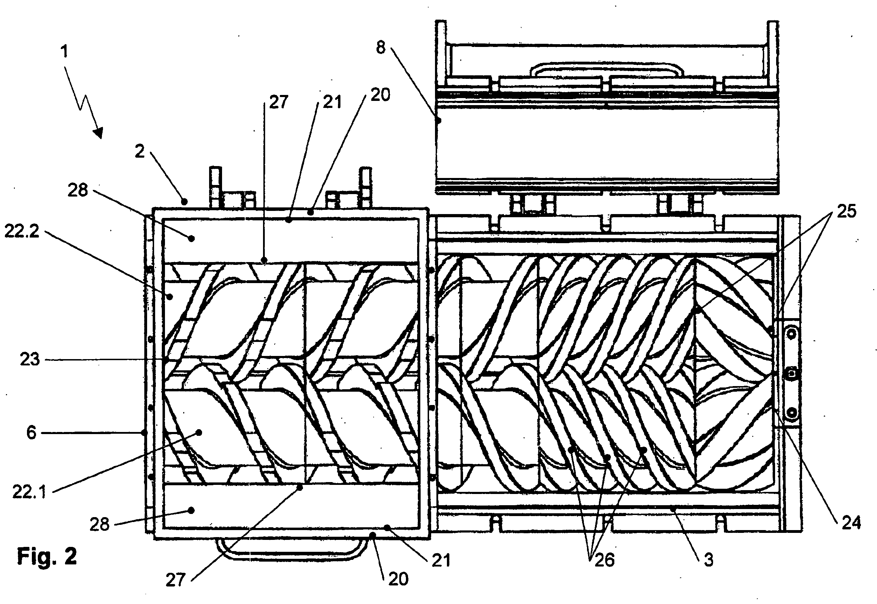

Die **Fig. 2** zeigt den Doppelschneckenextruder 1 in der Draufsicht. Dabei sind die zwei nebeneinander parallel angeordneten, gegenläufigen Extruderschnecken 22.1, 22.2 nur an einem ersten Ende 23 an der hinteren Stirnplatte 6 des länglichen Extrudergehäuses 2 gelagert und weisen am gegenüberliegenden zweiten Ende 24 jeweils ein nichtgelagertes, freies Endmodul 25 auf, das zu dem Teilbereich gehört, der im Schließzustand vom hohlzylinderförmigen Führungsabschnitt aus dem Gehäuseunterteil 3 und dem Gehäusedeckel 8 umschlossen wird. Die Extruderschnecken 22.1, 22.2 weisen jeweils eine Schneckensteigung auf, die vom ersten Ende 23 bis zum Beginn des freien Endmoduls 25 kleiner wird, wobei das freie Endmodul 25 eine gegenläufige Schneckensteigung aufweist. Eine kleinere Schneckensteigung ist gleichbedeutend mit einem kleineren Abstand zwischen den Stegen 26 der Schneckenwendel. Wie **Fig. 2** ebenfalls zeigt, weist der Einlauftrichter 20 neben der rechteckigen, oberen Eintrittsöffnung 21 eine schmalere rechteckige, untere Austrittsöffnung 27 auf, wobei die Eintrittsöffnung 21 und die Austrittsöffnung 27 durch zwei an den Längsseiten des Extrudergehäuses 2 gegenüberliegende nach innen schräg abfallende Trichterwände 28 verbunden sind. Dabei befindet sich die untere Austrittsöffnung 27 oberhalb der Extruderschnecken 22.1, 22.2 und ist auf diese gerichtet, wodurch das zu extrudierende Material direkt auf die Extruderschnecken 22.1, 22.2 gegeben werden kann. Die **Fig. 1** und die **Fig. 2** dienen lediglich der Illustration der Anordnung der Extruderschnecken 22.1, 22.2 innerhalb des Doppelschneckenextruders 1 und zeigen dabei nicht die erfindungsgemäße Ausbildung der Schneckensteigung.

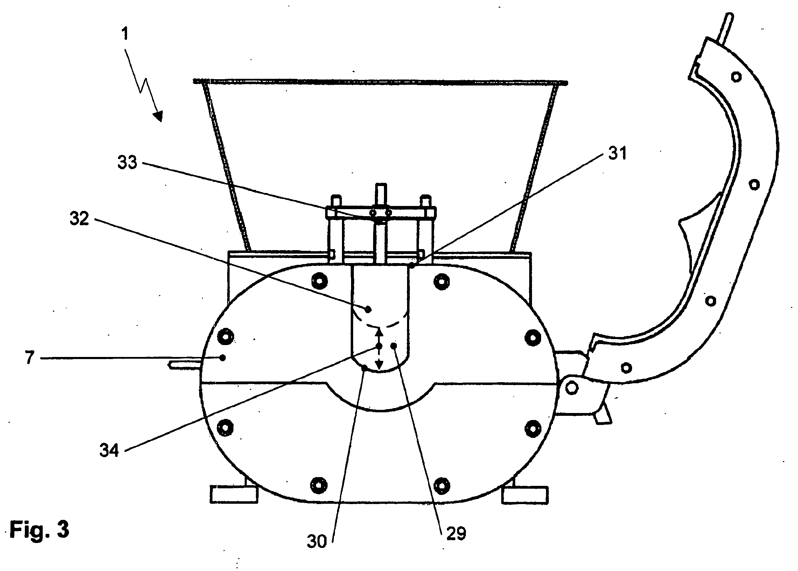

Die **Fig. 3** zeigt eine Vorderansicht des Doppelschneckenextruders 1. Die vordere Stirnplatte 7 weist eine größenverstellbare Auslauföffnung 29 zur Mengendosierung des extrudierten Materials auf. Dafür ist eine Schieberausnehmung 30 in der vorderen Stirnplatte 7 vorgesehen, die sich vom oberen Rand 31 der Stirnplatte 7 senkrecht nach unten bis etwa in den mittleren Bereich der Stirnplatte 7 erstreckt, wobei die Schieberausnehmung 30 im mittleren Bereich konkav abgerundet ist. Gemäß der Darstellung in **Fig. 3** ist die Schieberausnehmung 30 durch ein mit der Schieberausnehmung 30 formgleiches Schieberformstück 32 abgedeckt. Oberhalb des Schieberformstückes 32 ist eine Stellspindel 33 angeordnet, die mit einer Kurbel bedient werden kann, wobei die Stellspindel 33 mit dem oberen Ende des Schieberformstücks 32 verbunden ist. Die Größe der Auslauföffnung 29 und somit die Mengendosierung des extrudierten Materials kann über die vertikale Schiebebewegung 34 des Schieberformstückes 32 innerhalb der formgleichen Schieberausnehmung 30 unter Betätigung der Stellspindel 33 erfolgen.

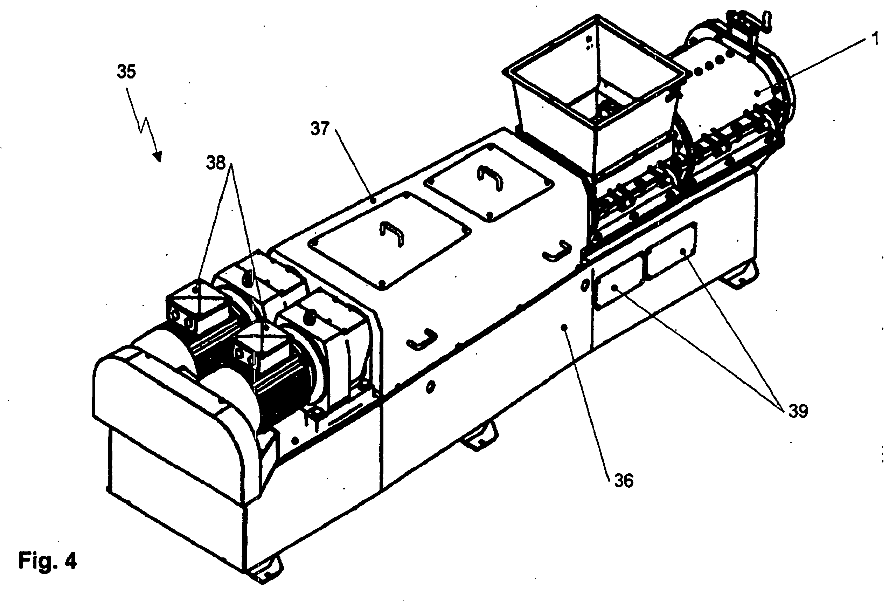

Die **Fig. 4** zeigt den oben beschriebenen Doppelschneckenextruder 1 als Teil einer Vorrichtung 35, umfassend
- ein Untergestell 36,
- ein Antriebsmodul 37, das auf dem Untergestell 36 befestigt ist, mit zwei Getriebemotoren 38 und zwei Kardangelenkwellen zur Kraftübertragung sowie zwei mit den Kardangelenkwellen verbundene Extruderachsen.

Gemäß **Fig. 4** ist der Doppelschneckenextruder 1 hinter dem Antriebsmodul 37 angeordnet, wobei die (nicht dargestellten) Extruderschnecken 22.1, 22.2 des Doppelschneckenextruders 1 durch das Antriebsmodul 37 angetrieben werden. Das Untergestell 36, auf dem der Doppelschneckenextruder 1 befestigt ist, weist unterhalb des Doppelschneckenextruders 1 zwei Zugangstüren 39 für die Wartung auf.

### LISTE DER BEZUGSZEICHEN

- 1: Doppelschneckenextruder
- 2: Extrudergehäuse
- 3: Gehäuseunterteil
- 4: Öffnung (des Gehäuseunterteils 3)
- 5.1: obere Längsrandfläche des Gehäuseunterteils 3
- 5.2: obere Längsrandfläche des Gehäuseunterteils 3
- 6: hintere Stirnplatte
- 7: vordere Stirnplatte
- 8: Gehäusedeckel
- 9: Öffnung (des Gehäusedeckels 8)
- 10.1: untere Längsrandfläche des Gehäusedeckels 8
- 10.2: untere Längsrandfläche des Gehäusedeckels 8
- 11: Verschleißeinlage (des Gehäuseunterteils 3)
- 12: Verschleißeinlage (des Gehäusedeckels 8)
- 13: Flansche (der Verschleißeinlage 11)
- 14: Flansche (der Verschleißeinlage 12)
- 15: flache Deckwandung
- 16: nach unten gewölbte Seitenwandungen
- 17: Führungsprisma/Führungsprismen
- 18: gekrümmte Schenkel (des Führungsprismas 17)
- 19: Gehäuseoberteil
- 20: Einlauftrichter
- 21: obere Eintrittsöffnung (des Einlauftrichters 20)
- 22.1: Extruderschnecken
- 22.2: Extruderschnecken
- 23: erstes Ende (der Extruderschnecken 22.1, 22.2)
- 24: zweites Ende (der Extruderschnecken 22.1, 22.2)
- 25: freies Endmodul
- 26: Stege der Schneckenwendel
- 27: untere Austrittsöffnung (des Einlauftrichters 20)
- 28: schräg abfallende Trichterwände
- 29: Auslauföffnung
- 30: Schieberausnehmung in der vorderen Stirnplatte 7
- 31: oberer Rand/Rand der vorderen Stirnplatte 7
- 32: Schieberformstück
- 33: Stellspindel
- 34: Schiebebewegung
- 35: Vorrichtung zum thermomechanischen Aufschluss von organischen Roh- und Reststoffen
- 36: Untergestell
- 37: Antriebsmodul
- 38: Getriebemotoren
- 39: Zugangstüren

## Patentansprüche

1. Doppelschneckenextruder (1) zum thermomechanischen Aufschluss von organischen Roh- und Reststoffen, umfassend
• ein längliches Extrudergehäuse (2), aufweisend ein Gehäuseunterteil (3) mit einem auf der Innenseite U-schalenförmigen Querschnitt mit nach oben gerichteter Öffnung (4), das sich horizontal zwischen einer hinteren Stirnplatte (6) und einer vorderen Stirnplatte (7) erstreckt, wobei das Gehäuseunterteil (3) in einem Schließzustand zusammen mit einem Gehäusedeckel (8), der einen auf der Innenseite umgedreht U-schalenförmigen Querschnitt mit nach unten gerichteter Öffnung (9) aufweist, durch Übereinanderlegen der Öffnung (4) des Gehäuseunterteils (3) und der Öffnung (9) des Gehäusedeckels (8) einen hohlzylinderartigen Führungsabschnitt bildet, der an der vorderen Stirnplatte (7) anliegt,
• zwei nebeneinander parallel angeordnete, gegenläufige Extruderschnecken (22.1, 22.2), die beide, jeweils freitragend, nur an einem ersten Ende (23) an der hinteren Stirnplatte (6) des länglichen Extrudergehäuses (2) gelagert sind und am gegenüberliegenden zweiten Ende (24) jeweils ein nichtgelagertes, freies Endmodul (25) aufweisen, wobei die Extruderschnecken (22.1, 22.2) im Schließzustand zumindest teilweise vom hohlzylinderförmigen Führungsabschnitt aus dem Gehäuseunterteil (3) und dem Gehäusedeckel (8) umschlossen sind,
wobei das Gehäuseunterteil (3) und der Gehäusedeckel (8) auf ihren Innenseiten jeweils mit Verschleißeinlagen (11, 12) versehen sind und die Verschleißeinlage (11) des Gehäuseunterteils (3) einen U-schalenförmigen Querschnitt mit nach oben gewölbten Seitenwandungen aufweist, die im U-schalenförmigen Querschnitt die U-Schenkel bilden und an ihren oberen Enden an der Öffnung (4) jeweils unter Bildung von Flanschen (13) nach außen horizontal abgekantet sind, und wobei die Verschleißeinlage (12) des Gehäusedeckels (8) einen im Schließzustand umgedreht U-schalenförmigen Querschnitt mit zwei nach unten gewölbten Seitenwandungen (16) aufweist, die bei dem im Schließzustand umgedreht U-schalenförmigen Querschnitt die U-Schenkel bilden und an ihren unteren Enden unter Bildung von Flanschen (14) nach außen horizontal abgekantet sind, und dass im Schließzustand beim Übereinanderlegen der Öffnungen (4, 9) die beiden nach außen abgekanteten Flansche (13) des Gehäuseunterteils (3) und die nach außen abgekanteten Flansche (14) des Gehäusedeckels (8) miteinander kontaktieren, **dadurch gekennzeichnet, dass** die Extruderschnecken (22.1, 22.2) jeweils eine Schneckensteigung aufweisen, die vom ersten Ende (23) bis zum Beginn des freien Endmoduls (25) immer kleiner wird, wobei eine kleinere Schneckensteigung gleichbedeutend ist mit einem kleineren Abstand zwischen den Stegen (26) der Schneckenwendel, und wobei das freie Endmodul (25) eine gegenläufige Schneckensteigung aufweist.

2. Doppelschneckenextruder (1) nach Anspruch 1, **dadurch gekennzeichnet, dass**
• die Verschleißeinlage (11) des Gehäuseunterteils (3) eine flache Bodenwandung, die im Querschnitt den U-Basisbereich bildet, aufweist, und
• die Verschleißeinlage (12) des Gehäusedeckels (8) eine flache Deckwandung (15), die bei dem im Schließzustand umgedreht U-schalenförmigen Querschnitt den U-Basisbereich bildet, aufweist,
wobei die flache Deckwandung (15) und die flache Bodenwandung im Schließzustand parallel beabstandet sind, und dass auf der flachen Bodenwandung der Verschleißeinlage (11) und auf der flachen Deckwandung (15) der Verschleißeinlage (12) jeweils ein Führungsprisma (17) ausgebildet ist, das im Querschnitt jeweils die Form eines dreieckigen, jeweils in der Mitte der Bodenwandung und der Deckwandung (15) platzierten Vorsprungs mit konkav gekrümmten Schenkeln (18) aufweist und sich über die gesamte Länge der jeweiligen Verschleißeinlage (11, 12) erstreckt.

3. Doppelschneckenextruder (1) nach Anspruch 1 oder 2, **dadurch gekennzeichnet, dass** die Verschleißeinlagen (11, 12) aus einem weichen Trägerblech bestehen, das mit einer Härteschicht aus hochverschleißfestem Verbundpanzermaterial überzogen ist.

4. Doppelschneckenextruder (1) nach einem der Ansprüche 1 bis 3, **dadurch gekennzeichnet, dass** auf dem Gehäuseunterteil (3) in einem Bereich, der sich zwischen der hinteren Stirnplatte (6) und dem Gehäusedeckel (8) erstreckt, ein Gehäuseoberteil (19) mit einem Einlauftrichter (20) mit einer oberen Eintrittsöffnung (21) und einer unteren Austrittsöffnung (27) vorgesehen ist, wobei sich die untere Austrittsöffnung (27) des Einlauftrichters (20) oberhalb der Extruderschnecken (22.1, 22.2) befindet und auf diese gerichtet ist.

5. Doppelschneckenextruder (1) nach Anspruch 4, **dadurch gekennzeichnet, dass** in einem Bereich zwischen dem Einlauftrichter (20) und dem Gehäusedeckel (8) die Extruderschnecken (22.1, 22.2) mit einer zum Gehäusedeckel (8) hin abfallenden Einlaufschräge überdacht sind.

6. Doppelschneckenextruder (1) nach einem der Ansprüche 1 bis 5, **dadurch gekennzeichnet, dass** die vordere Stirnplatte (7) eine größenverstellbare Auslauföffnung (29) zur Mengendosierung des extrudierten Materials aufweist, wobei eine von einem Rand (31) der vorderen Stirnplatte (7) ausgehende Schieberausnehmung (30) in der vorderen Stirnplatte (7) vorgesehen ist und eine Regelung der Größe der Auslauföffnung (29) über die Schiebebewegung (34) eines mit der Schieberausnehmung (30) formgleichen Schieberformstückes (32) innerhalb der Schieberausnehmung (30) mittels einer mit dem Schieberformstück (32) verbundenen Stellspindel (33) erfolgen kann.

7. Vorrichtung (35) zum thermomechanischen Aufschluss von organischen Roh-und Reststoffen, umfassend
▪ ein Untergestell (36),
▪ ein Antriebsmodul (37), das auf dem Untergestell (36) befestigt ist, mit zwei Getriebemotoren (38) und zwei Kardangelenkwellen zur Kraftübertragung sowie zwei mit den Kardangelenkwellen verbundenen Extruderachsen,
▪ einen Doppelschneckenextruder (1) nach einem der Ansprüche 1 bis 6, der auf dem Untergestell (36) hinter dem Antriebsmodul (37) angeordnet ist, wobei die Extruderschnecken (22.1, 22.2) des Doppelschneckenextruders (1) durch das Antriebsmodul (37) angetrieben werden,
wobei das Untergestell (36), auf dem der Doppelschneckenextruder (1) befestigt ist, unterhalb des Doppelschneckenextruders (1) Zugangstüren (39) für die Wartung aufweist.

8. Verfahren zum thermomechanischen Aufschluss von organischen Roh- und Reststoffen, bei dem das aufzuschließende Material einem Doppelschneckenextruder (1) zugeführt wird, in dem das aufzuschließende Material einen Extrusionsprozess durchläuft, wobei der Doppelschneckenextruder (1) Folgendes umfasst:
• ein längliches Extrudergehäuse (2), aufweisend ein Gehäuseunterteil (3) mit einem auf der Innenseite U-schalenförmigen Querschnitt mit nach oben gerichteter Öffnung (4), das sich horizontal zwischen einer hinteren Stirnplatte (6) und einer vorderen Stirnplatte (7) erstreckt, wobei das Gehäuseunterteil (3) in einem Schließzustand, der während des Extrusionsprozesses vorliegt, zusammen mit einem Gehäusedeckel (8), der einen auf der Innenseite umgedreht U-schalenförmigen Querschnitt mit nach unten gerichteter Öffnung (9) aufweist, durch Übereinanderlegen der Öffnung (4) des Gehäuseunterteils (3) und der Öffnung (9) des Gehäusedeckels (8) einen hohlzylinderartigen Führungsabschnitt bildet, der an der vorderen Stirnplatte (7) anliegt,
• zwei nebeneinander parallel angeordnete, gegenläufige Extruderschnecken (22.1, 22.2), die beide, jeweils freitragend, nur an einem ersten Ende (23) an der hinteren Stirnplatte (6) des länglichen Extrudergehäuses (2) gelagert sind und am gegenüberliegenden zweiten Ende (24) jeweils ein nichtgelagertes, freies Endmodul (25) aufweisen, wobei die Extruderschnecken (22.1, 22.2) im Schließzustand während des Extrusionsprozesses zumindest teilweise vom hohlzylinderförmigen Führungsabschnitt aus dem Gehäuseunterteil (3) und dem Gehäusedeckel (8) umschlossen sind,
wobei das Gehäuseunterteil (3) und der Gehäusedeckel (8) auf ihren Innenseiten jeweils mit Verschleißeinlagen (11, 12) versehen sind und die Verschleißeinlage (11) des Gehäuseunterteils (3) einen U-schalenförmigen Querschnitt mit nach oben gewölbten Seitenwandungen aufweist, die im U-schalenförmigen Querschnitt die U-Schenkel bilden und an ihren oberen Enden an der Öffnung (4) jeweils unter Bildung von Flanschen (13) nach außen horizontal abgekantet sind, und wobei die Verschleißeinlage (12) des Gehäusedeckels (8) einen im Schließzustand während des Extrusionsprozesses umgedreht U-schalenförmigen Querschnitt mit zwei nach unten gewölbten Seitenwandungen (16) aufweist, die bei dem im Schließzustand während des Extrusionsprozesses umgedreht U-schalenförmigen Querschnitt die U-Schenkel bilden und an ihren unteren Enden unter Bildung von Flanschen (14) nach außen horizontal abgekantet sind, und dass im Schließzustand beim Übereinanderlegen der Öffnungen (4, 9) die beiden nach außen abgekanteten Flansche (13) des Gehäuseunterteils (3) und die nach außen abgekanteten Flansche (14) des Gehäusedeckels (8) miteinander kontaktieren, **dadurch gekennzeichnet, dass** die Extruderschnecken (22.1, 22.2) jeweils eine Schneckensteigung aufweisen, die vom ersten Ende (23) bis zum Beginn des freien Endmoduls (25) immer kleiner wird, wobei eine kleinere Schneckensteigung gleichbedeutend ist mit einem kleineren Abstand zwischen den Stegen (26) der Schneckenwendel, und wobei das freie Endmodul (25) eine gegenläufige Schneckensteigung aufweist.

9. Verfahren nach Anspruch 8, **dadurch gekennzeichnet, dass** das zu extrudierende Material über eine externe Zuführeinrichtung in einen Einlauftrichter (20) gelangt, der direkt zu den gegenläufigen Extruderschnecken (22.1, 22.2) führt, und dass der Durchfluss des Materials über eine größenverstellbare Auslassöffnung (29) an der vorderen Stirnplatte (7) des Doppelschneckenextruders (1) geregelt wird.

10. Verfahren nach Anspruch 8 oder 9, **dadurch gekennzeichnet, dass** das zu extrudierende Material Biomasse ist, die vor der Zuführung in eine anaerobe Fermentationsanlage zur Erzeugung von Biogas thermomechanisch aufgeschlossen wird.

## Claims

1. Twin-screw extruder (1) for thermomechanical pulping of organic raw materials and residues, comprising:
• an elongated extruder barrel (2) having a lower barrel part (3) with a U-cup-shaped cross-section on the inside with an upwardly directed opening (4), extending horizontally between a rear end plate (6) and a front end plate (7), wherein the lower barrel part (3) in a closed state together with a barrel cover (8), having an inversed U-cup-shaped cross-section on the inside with a downwardly directed opening (9), by superposing the opening (4) of the lower barrel part (3) and the opening (9) of the barrel cover (8) forms a hollow-cylindrical guide portion which abuts against the front end plate (7),
• two counter-directional extruder screws (22.1, 22.2), being arranged side by side in parallel and being in each case self-supporting, are mounted only at a first end (23) on the rear end plate (6) of the elongated extruder barrel (2) and at the opposite second end (24) each feature a non-mounted free end module (25), wherein said extruder screws (22.1, 22.2) in the closed state are at least partially enclosed by the hollow-cylindrical guide portion of the lower barrel part (3) and the barrel cover (8),
wherein the lower barrel part (3) and the barrel cover (8) on their insides are each provided with wear inserts (11, 12), said wear insert (11) of the lower barrel part (3) having a U-cup-shaped cross-section with upwardly curved side walls, which in the U-cup-shaped cross-section form the U-legs and at their upper ends at the opening (4) in each case are beveled horizontally outwardly while forming flanges (13), and wherein the wear insert (12) of the barrel cover (8) in the closed state features an inverted U-cup-shaped cross-section with two downwardly curved side walls (16) forming the U-legs in the inverted U-cup-shaped cross-section in the closed state and at their lower ends being horizontally beveled outwardly while forming flanges (14), and in the closed position, when superposing the openings (4, 9), the two outwardly beveled flanges (13) of the lower barrel part (3) and the outwardly beveled flanges (14) of the barrel cover (8) contact one another, **characterized in that** the extruder screws (22.1, 22.2) each have a screw pitch which becomes increasingly smaller from the first end (23) towards the beginning of the free end module (25), wherein a smaller screw pitch is equivalent to a smaller distance between the webs (26) of the screw pitch, and wherein the free end module (25) features a counter-directional screw pitch.

2. Twin-screw extruder (1) according to Claim 1, **characterized in that**
• the wear insert (11) of the lower barrel part (3) has a flat bottom wall which in the cross-section forms the U-base portion, and
• the wear insert (12) of the barrel cover (8) has a flat top wall (15) which in the inverted U-cup-shaped cross-section in the closed state forms the U-base portion,
wherein the flat top wall (15) and the flat bottom wall in the closed state are spaced apart in parallel, and a guide prism (17) is each formed on the flat bottom wall of the wear insert (11) and on the flat top wall (15) of the wear insert (12), and in the cross-section respectively has the shape of a triangular protrusion having concavely curved legs (18) and being in each case placed in the center of the bottom wall and the top wall (15), and extending over the entire length of the respective wear insert (11, 12).

3. Twin-screw extruder (1) according to claim 1 or 2, **characterized in that** the wear inserts (11, 12) consist of a flexible support plate which is coated with a hardened layer of highly wear-resistant composite armor material.

4. Twin-screw extruder (1) according to any of claims 1 to 3, **characterized in that** an upper barrel part (19) with a feed hopper (20) having an upper inlet opening (21) and a lower outlet opening (27) is provided in a region extending between the rear end plate (6) and the barrel cover (8), wherein the lower outlet opening (27) of the feed hopper (20) is located above and directed to the extruder screws (22.1, 22.2).

5. Twin-screw extruder (1) according to claim 4, **characterized in that** the extruder screws (22.1, 22.2) in a region between the feed hopper (20) and the barrel cover (8) are covered with an infeed bevel sloping down towards the barrel cover (8).

6. Twin-screw extruder (1) according to any of claims 1 to 5, **characterized in that** the front end plate (7) has a size-adjustable outlet opening (29) for quantity metering of the extruded material, wherein a slide recess (30) emanating from an edge (31) of the front end plate (7) is provided in the front end plate (7), and adjustment of the size of the outlet opening (29) can be realized via the sliding movement (34) of a slide fitting (32) of the same shape with the slide recess (30) within the slide recess (30) by means of an adjusting spindle (33) being connected to the slide fitting (32).

7. Device (35) for thermomechanical pulping of organic raw materials and residues comprising:
• a base frame (36),
• a drive module (37) which is mounted on the base frame (36), comprising two gear motors (38) and two cardan shafts for transmitting power, and two extruder axes being connected to the cardan shafts,
• a twin-screw extruder (1) according to any of claims 1 to 6, which is arranged on the base frame (36) behind the drive module (37), wherein the extruder screws (22.1, 22.2) of the twin-screw extruder (1) are driven by the drive module (37),
**characterized in that** the base frame (36) on which the twin-screw extruder (1) is mounted has access doors (39) for maintenance below the twin-screw extruder (1).

8. Method for thermomechanical pulping of organic raw materials and residues, wherein the material to be digested is supplied to a twin-screw extruder (1) in which the material to be digested passes through an extrusion process, wherein the twin-screw extruder (1) comprises the following:
• an elongated extruder barrel (2) having a lower barrel part (3) with a U-cup-shaped cross-section on the inside with an upwardly directed opening (4), extending horizontally between a rear end plate (6) and a front end plate (7), wherein the lower barrel part (3) in a closed state, which is present during the extrusion process, together with a barrel cover (8) having an inverted U-cup- shaped cross-section on the inside with a downwardly directed opening (9), by superposing the opening (4) of the lower barrel part (3) and the opening (9) of the barrel cover (8) forms a hollow-cylindrical guide portion which abuts against the front end plate (7),
• two counter-directional extruder screws (22.1, 22.2), being arranged side by side in parallel and being in each case self-supporting, are mounted only at a first end (23) at the rear end plate (6) of the elongated extruder barrel (2) and at the opposite second end (24) each feature a non-mounted free end module (25), wherein the extruder screws (22.1, 22.2) in the closed state during the extrusion process are at least partially enclosed by the hollow-cylindrical guide portion of the lower barrel part (3) and the barrel cover (8),
wherein the lower barrel part (3) and the barrel cover (8) on their insides are each provided with wear inserts (11, 12), said wear insert (11) of the lower barrel part (3) having a U-cup-shaped cross-section with upwardly curved side walls, which in the U-cup-shaped cross-section form the U-legs and at their upper ends at the opening (4) in each case are horizontally beveled outwardly while forming flanges (13), and wherein the wear insert (12) of the barrel cover (8) in the closed state during the extrusion process features an inverted U-cup-shaped cross-section with two downwardly curved side walls (16) forming the U-legs in the inverted U-cup-shaped cross-section in the closed state during the extrusion process and at their lower ends being horizontally beveled outwardly while forming flanges (14), and in the closed state, when superposing the openings (4, 9), the two outwardly beveled flanges (13) of the lower barrel part (3) and the outwardly beveled flanges (13) of the barrel cover (8) contact one another, **characterized in that** the extruder screws (22.1, 22.2) each feature a screw pitch becoming increasingly smaller from the first end (23) towards the beginning of the free end module (25), wherein a smaller screw pitch is equivalent to a smaller distance between the webs (26) of the screw pitch, and wherein the free end module (25) features a counter-directional screw pitch.

9. Method according to claim 8, **characterized in that** the material to be extruded passes from an external feeder to a feed hopper (20) which leads directly to the counter-directional extruder screws (22.1, 22.2), and the flow of material is regulated via a size-adjustable outlet opening (29) at the front end plate (7) of the twin-screw extruder (1).

10. Method according to claim 8 or 9, **characterized in that** the material to be extruded is biomass which is thermomechanically digested before being supplied to an anaerobic fermentation system for production of biogas.

## Revendications

1. Extrudeuse à vis jumelées (1) pour le dépulpage thermomécanique de matières premières organiques et de résidus, comprenant:
• un carter d'extrudeuse allongé (2) présentant une partie inférieure du carter (3) avec une section transversale en forme de coupe en U à l'intérieur avec une ouverture (4) étant dirigée vers le haut, s'étendant horizontalement entre une plaque terminale arrière (6) et une plaque terminale avant (7), dans lequel la partie inférieure du carter (3), dans un état fermé, conjointement avec un couvercle du carter (8), présentant une section transversale inversée en forme de coupe en U à l'intérieur avec une ouverture (9) étant dirigée vers le bas, en superposant l'ouverture (4) de la partie inférieure du carter (3) et l'ouverture (9) du couvercle du carter (8), forme une section de guidage en forme de cylindre creux venant en butée contre la plaque terminale avant (7),
• deux vis d'extrudeuse (22.1, 22.2) conçues en sens inversé étant agencées l'une à côté de l'autre en parallèle et étant dans chaque cas autoportante, sont montées seulement à une première extrémité (23) sur la plaque terminale arrière (6) du carter d'extrudeuse allongé (2), et à la deuxième extrémité opposée (24) présentent chacune un module terminale libre (25) non monté, dans lequel lesdites vis d'extrudeuse (22.1, 22.2), dans l'état fermé, sont au moins partiellement entourées par la section de guidage en forme de cylindre creux de la partie inférieure du carter (3) et le couvercle du carter (8),
dans laquelle la partie inférieure du carter (3) et le couvercle du carter (8) à leur intérieur sont chacun muni d'inserts d'usure (11, 12), ledit insert d'usure (11) de la partie inférieure du carter (3) présentant une section transversale en forme de coupe en U avec des parois latérales courbées vers le haut formant les jambes en forme de U dans la section transversale en forme de coupe en U, et à leurs extrémités supérieures à l'ouverture (4) dans chaque cas étant chanfreinées horizontalement vers l'extérieur en formant des flasques (13), et dans laquelle l'insert d'usure (12) du couvercle du carter (8), dans l'état fermé, présente une section transversale inversée en forme de coupe en U avec deux parois latérales (16) courbées vers le bas formant les jambes en forme de U dans la section transversale inversée en forme de coupe en U dans l'état fermé, et à leurs extrémités inférieures étant chanfreinées horizontalement vers l'extérieur en formant des flasques (14), et dans l'état fermé, en superposant les ouvertures (4, 9), les deux flasques (13) étant chanfreinés vers l'extérieur de la partie inférieure du carter (3) et les flasques (14) étant chanfreinés vers l'extérieur du couvercle du carter (8) prennent contact l'une avec l'autre, **caractérisée en ce que** les vis d'extrudeuse (22.1, 22.2) présentent chacune un pas de vis devenant de plus en plus petit à partir de la première extrémité (23) vers le début du module terminale libre (25), dans lequel un pas de vis plus petit équivaut à une distance plus petite entre les âmes (26) du pas de vis, et dans lequel le module terminale libre (25) présente un pas de vis en sens inversé.

2. Extrudeuse à vis jumelées (1) selon la revendication 1, **caractérisée en ce que**
• l'insert d'usure (11) de la partie inférieure du carter (3) présente une paroi de fond plate formant dans la section transversale la section de base en forme de U, et
• l'insert d'usure (12) du couvercle du carter (8) présente une paroi de plafond plate (15) formant, dans l'état fermé, la section de base en forme de U dans la section transversale inversée en forme de coupe en U,
dans laquelle la paroi de plafond plate (15) et la paroi de fond plate, dans l'état fermé, sont espacées l'une de l'autre en parallèle, et un prisme de guidage (17) est formé respectivement sur la paroi de fond plate de l'insert d'usure (11) et sur la paroi de plafond plate (15) de l'insert d'usure (12) et dans la section transversale présente la forme d'une saillie triangulaire ayant des jambes courbées de façon concave (18) et étant dans chaque cas placées au millieu de la paroi de fond et de la paroi de plafond (15), et s'étendant sur toute la longueur de l'insert d'usure (11, 12) correspondant.

3. Extrudeuse à vis jumelées (1) selon la revendication 1 ou 2, **caractérisée en ce que** les inserts d'usure (11, 12) se composent d'une plaque de soutien flexible étant revêtue d'une couche durcie d'un matériau composite d'armure étant hautement résistant à l'usure.

4. Extrudeuse à vis jumelées (1) selon l'une quelconque des revendications 1 à 3, **caractérisée en ce qu'**une partie supérieure du carter (19) avec une trémie d'alimentation (2) présentant une ouverture d'entrée supérieure (21) et une ouverture de sortie inférieure (27) est pourvue dans une région s'étendant entre la plaque terminale arrière (6) et le couvercle du carter (8), dans laquelle l'ouverture de sortie inférieure (27) de la trémie d'alimentation (20) est agencée au-dessus de et est dirigée vers les vis d'extrudeuse (22.1, 22.2).

5. Extrudeuse à vis jumelées (1) selon la revendication 4, **caractérisée en ce que** les vis d'extrudeuse (22.1, 22.2), dans une région entre la trémis d'alimentation (20) et le couvercle du carter (8), sont recouvertes d'un chanfrein d'entree descendant vers le couvercle du carter (8).

6. Extrudeuse à vis jumelées (1) selon l'une quelconque des revendications 1 à 5, **caractérisée en ce que** la plaque terminale avant (7) présente une ouverture de sortie à taille réglable (29) pour la mésure de la quantité du matériau extrudé, dans laquelle un évidement de glissement (30) émanant à partir d'un bord (31) de la plaque terminale avant (7) est pourvue dans la plaque terminale avant (7), et le réglage de la taille de l'ouverture de sortie (29) peut être réalisé par le mouvement glissant (34) d'une pièce de façonnage glissante (32) ayant la même forme que l'évidement de glissement (30) dans l'évidement de glissement (30) par l'intermédiaire d'une broche de réglage (33) étant reliée à la pièce de façonnage glissante (32).

7. Dispositif (35) pour le dépulpage thermomécanique de matières premières organiques et de résidus, comprenant:
• un cadre de base (36),
• un module d'entraînement (37) étant monté sur le cadre de base (36), comprenant deux moteurs à engrenage (38) et deux arbres à cardan pour la transmission de puissance, et deux axes d'extrudeuse étant reliés aux arbres à cardan,
• une extrudeuse à vis jumelées (1) selon l'une quelconque des revendications 1 à 6, qui est agencée sur le cadre de base (36) derrière le module d'entraînement (37), dans laquelle les vis d'extrudeuse (22.1, 22.2) de l'extrudeuse à vis jumelées (1) sont entraînées par le module d'entraînement (37),
**caractérisé en ce que** le cadre de base (36), sur lequel l'extrudeuse à vis jumelées (1) est montée, présente des portes d'accès (39) destinées à l'entretien au-dessous de l'extrudeuse à vis jumelées (1).

8. Procédé pour le dépulpage thermomécanique de matières premières organiques et de résidus, dans lequel le matériau à digester est alimenté vers une extrudeuse à vis jumelées (1) dans laquelle le matériau à digester passe par un processus d'extrusion, dans lequel l'extrudeuse à vis jumelées (1) comprend le suivant:
• un carter d'extrudeuse allongé (2) présentant une partie inférieure du carter (3) avec une section transversale en forme de coupe en U à l'intérieur avec une ouverture (4) étant dirigée vers le haut, s'étendant horizontalement entre une plaque terminale arrière (6) et une plaque terminale avant (7), dans lequel la partie inférieure du carter (3), dans un état fermé étant présent dans le processus d'extrusion, conjointement avec un couvercle du carter (8) présentant une section transversale inversée en forme de coupe en U à l'intérieur avec une ouverture (9) étant dirigée vers le bas, en superposant l'ouverture (4) de la partie inférieure du carter (3) et l'ouverture (9) du couvercle du carter (8), forme une section de guidage en forme de cylindre creux venant en butée contre la plaque terminale avant (7),
• deux vis d'extrudeuse (22.1, 22.2) conçues en sens inversé étant agencées l'une à côté de l'autre en parallèle et étant dans chaque cas autoportante, sont montées seulement à une première extrémité (23) sur la plaque terminale arrière (6) du carter d'extrudeuse allongé (2) et à la deuxième extrémité opposée (24) présentent chacune un module terminale libre (25) non monté, dans lequel lesdites vis d'extrudeuse (22.1, 22.2), dans l'état fermé pendant le processus d'extrusion, sont au moins partiellement entourées par la section de guidage en forme de cylindre creux de la partie inférieure du carter (3) et le couvercle du carter (8),
dans laquelle la partie inférieure du carter (3) et le couvercle du carter (8) à l'intérieur sont chacun muni d'inserts d'usure (11, 12), ledit insert d'usure (11) de la partie inférieure du carter (3) présentant une section transversale en forme de coupe en U avec des parois latérales coubées vers le haut formant les jambes en forme de U dans la section transversale en forme de coupe en U et à leurs extrémités supérieures à l'ouverture (4) dans chaque cas étant chanfreinées horizontalement vers l'extérieur en formant de flasques (13), et dans laquelle l'insert d'usure (12) du couvercle du carter (8), dans l'état fermé pendant le processus d'extrusion, présente une section transversale inversée en forme de coupe en U avec deux parois latérales (16) courbées vers le bas formant les jambes en forme de U dans la section transversale inversée en forme de coupe en U dans l'état fermé pendant le processus d'extrusion, et à leurs extrémités inférieures étant chanfreinées horizontalement vers l'extérieur en formant des flasques (14), et dans l'état fermé, en superposant les ouvertures (4, 9), les deux flasques (13) étant chanfreinés vers l'extérieur de la partie inférieure du carter (3) et les flasques (13) étant chanfreinés vers l'éxtérieur du couverture du carter (8) prennent contact l'un avec l'autre, **caractérisée en ce que** les vis d'extrudeuse (22.1, 22.2) présentent chacune un pas de vis devenant de plus en plus petit à partir de la première extrémité (23) vers le début du module terminale libre (25), dans lequel un pas de vis plus petit équivaut à une distance plus petite entre les âmes (26) du pas de vis, et dans lequel le module terminale libre (25) présente un pas de vis en sens inversé.

9. Procédé selon la revendication 8, **caractérisé en ce que** le matériau à extruder passe d'un système externe d'alimentation jusqu'à une trémie d'alimentation (20) mènant directement aux vis d'extrudeuse (22.1, 22.2) en sens inversé, et le flux de matériau étant regulé par une overture de sortie (29) à taille reglable à la plaque terminale avant (7) de l'extrudeuse à vis jumelées (1).

10. Procédé selon la revendication 8 ou 9, **caractérisé en ce que** le matériau à extruder est de la biomasse étant thermomécaniquement digestée avant d'être alimentée jusqu'à un système de fermentation anaérobie pour la production de biogaz.
